# EUROPÄISCHE PATENTSCHRIFT

(11) **EP 1 632 521 B1**
(45) Veröffentlichungstag und Bekanntmachung des Hinweises auf die Patenterteilung: **26.03.2008**
(21) Anmeldenummer: 05014232.2
(22) Anmeldetag: 30.06.2005
(51) Int. Cl.: C08J 3/03, C04B 41/49

(54) **Öl-in-Wasser-Emulsionen von Aminosiloxanen**
Oil in water emulsions of aminosiloxanes
Emulsions des aminosiloxanes du type huile-dans-eau

(30) Priorität: 19.08.2004 DE 102004040266
(43) Veröffentlichungstag der Anmeldung: 08.03.2006
(73) Patentinhaber: Wacker Chemie AG, 81737 München (DE)
(72) Erfinder: Mahr, Günther, Dr., 84489 Burghausen (DE); Burger, Willibald, Dr., 84489 Burghausen (DE); Esterbauer, Ludwig, 84539 Ampfing (DE); Wimmer, Franz, X., 84489 Burghausen (DE)
(74) Vertreter: Fritz, Helmut

(56) Entgegenhaltungen:
- EP-A- 0 186 265
- EP-A- 0 242 798
- EP-A- 0 556 740
- US-B1- 6 294 608

## Beschreibung

Die Erfindung betrifft die Verwendung von Öl-in-wasser Emulsionen von Aminoalkylgruppen aufweisendem Polydimethylsiloxan zur Behandlung von Leder und für die Papierleimung.

Solvensbasierende Zusammensetzungen auf Basis aminofunktioneller Polydimethylsiloxane, die z. B. als wasserabweisende Pflegemittel eingesetzt werden, sind bekannt.

US 6596060, Atofina, P. Michaud, beschreibt eine wasserabweisende Zusammensetzung, die im Wesentlichen aus mindestens einem Organopolysiloxan mit mindestens einer aminierten Gruppe und 85-99 Massen-% an Isopropanol besteht. Solvensbasierende Zusammensetzungen auf Basis von (aminofunktionellen) Organopolysiloxanen zeichnen sich im Allgemeinen durch eine sehr gute hydrophobierende Wirkung auf unterschiedlichsten Substraten aus, sind aber in Zeiten eines gestiegenen Umweltbewußtseins in Bezug auf flüchtige organische Verbindungen (VOCs) und aus gesundheitlicher Sicht nicht mehr zeitgemäß. Wässerige Zusammensetzungen werden deshalb in der Zukunft solvensbasierende Systeme generell mehr und mehr ablösen.

Wässerige Zusammensetzungen, die aminofunktionelle Organopolysiloxane enthalten, sind bekannt. Derartige Zusammensetzungen werden beispielsweise zur hydrophobierenden Behandlung von natürlichen und/oder synthetischen textilen Fasern, zur hydrophobierenden Behandlung von Baustoffen, als Bestandteil von Pflegemitteln und als Bestandteil von kosmetischen Zubereitungen eingesetzt. Herkömmliche wässerige Zusammensetzungen, die aminofunktionelle Organopolysiloxane enthalten, sind dem Fachmann in Form von sogenannten Öl-in-Wasser- oder Wasser-in-Öl-Emulsionen bekannt. Diese Emulsionen können in Form von sogenannten Makro- oder Mikroemulsionen vorliegen. Diese Emulsionen sind in der Regel durch herkömmliche nichtionogene, anionische, kationische oder amphotere Emulgatoren oder Silicon-Polyether-Copolymer-Emulgatoren stabilisiert. DE 4328917, Wacker-Chemie GmbH, M. Geck, beschreibt ein Verfahren zur Herstellung von Microemulsionen auf Basis aminofunktioneller Organopolysiloxane, bei dem die Komponenten Organopolysiloxan, herkömmlicher Emulgator, Wasser, gegebenenfalls Cotensid und gegebenenfalls Säure in beliebiger Reihenfolge zusammengegeben und vermischt werden.

Die Anwesenheit von nichtionischen, anionischen, kationischen oder amphoteren Emulgatoren in diesen wässerigen Zusammensetzungen, die aminofunktionelle Organopolysiloxane enthalten, verschlechtert jedoch die hydrophobierenden Eigenschaften derartiger Emulsionen in der Applikation erheblich, mitunter sogar drastisch.

Wässerige Zusammensetzungen, die aminofunktionelle Organopolysiloxane enthalten und auf die Verwendung der o. g. herkömmlichen Emulgatoren verzichten, sind bekannt.

GB 1199501 beschreibt Hydrophobierungsmittel, insbesondere für Glasoberflächen, welche Isopropanol, Wasser und ein Umsetzungsprodukt eines hydroxiterminierten, basischen Stickstoff aufweisenden Organopolysiloxans mit organischen oder anorganischen Säuren enthalten. Die wässerigen Verdünnungen dieser Hydrophobierungsmittel weisen jedoch keine ausreichende Wirkstoffstabilität auf.

EP 1008616 A beschreibt w/o-Emulsionen, die aminofunktionelle Organopolysiloxane enthalten. Derartige Emulsionen weisen den Nachteil auf, daß sie eine vorgegebene Zusammensetzung aufweisen und mit Wasser nicht verdünnbar sind.

EP 1031593 A beschreibt eine Zusammensetzung, die aus einem Gemisch wasserlöslicher, aminofunktioneller und im Wesentlichen alkoxygruppenfreier Siliciumverbindungen, Wasser, gegebenenfalls Alkohol und gegebenenfalls Säure besteht. Aufgrund der verwendeten wasserlöslichen (Teil-) Kondensate von wasserlöslichen Aminosilanen ist die hydrophobierende Wirkung der Zusammensetzung stark eingeschränkt.

In EP 186265 A sowie EP 68671 A werden Zusammensetzungen beschrieben, die (1) eine Mischung eines (A) Salzes eines Organopolysiloxans mit mindestens einer Aminogruppe sowie mindestens einer reaktiven Gruppe -OX, wobei X Wasserstoffatom, Alkylrest oder Alkoxialkylrest bedeutet, mit einer (B) in (2) löslichen, mindestens zwei reaktive Gruppen -OX aufweisenden Organosiliciumverbindung und (2) ein wasserlösliches Lösungsmittel enthalten. Die wässerigen Verdünnungen dieser Zusammensetzungen weisen keine ausreichende Wirkstoffstabilität auf.

EP 242798 A beschreibt wässerige Emulsionen, die als Emulgiermittel das Salz von wasserlöslicher organischer oder anorganischer Säure und Polysiloxan, das zusätzlich zu anderen Silioxaneinheiten solche Siloxaneinheiten enthält, die einwertige, SiC-gebundene Reste mit basischem Stickstoff in Mengen von mindestens 0.5 Gewichtsprozent basischem Stickstoff, bezogen auf das Gewicht des Polysiloxans, aufweisen, enthalten. Wie bekannt ist, sind derartige Emulsionen nicht über einen längeren Zeitraum verdünnungsstabil.

EP 556740 A beschreibt Organopolysiloxanzusammensetzungen, die ein Salz aus einer wasserlöslichen organischen oder anorganischen Säure und einem Organopolysiloxan, welches mindestens einen SiC-gebundenen organischen Rest mit basischem Stickstoff aufweist, enthalten. Derartige Zusammensetzungen sind in Verdünnung wirkstoffstabil, jedoch in ihrer hydrophobierenden Wirkung nicht ausreichend.

In US 2004/0029981 A ist die Behandlung von Fasern mit emulgatorfreien Emulsionen von aminofunktionellen Siliconölen beschrieben.

Aufgabe der Erfindung ist es,wässrige Zusammensetzungen zu finden, die die oben genannten Nachteile nicht aufweisen.

Gegenstand der Erfindung ist die Verwendung von Öl-in-Wasser Emulsionen, die
(i) 100 Gew.-Teile eines Aminoalkylgruppen aufweisenden Polydimethylsiloxans (P) mit einer Aminzahl von mindestens 0,1 mequiv/g Polydimethylsiloxan (P), aufgebaut aus Einheiten der allgemeinen Formel I

   R¹ₐR²_{b}SiO(_{4-a-b/2}) (I),

   in der
   - **R¹**: gegebenenfalls mit Halogenen substituierte Alkylreste mit 1-40 Kohlenstoffatomen, Reste -OR oder -OH,
   - **R**: gegebenenfalls mit Halogenen substituierte Alkylreste mit 1-40 Kohlenstoffatomen,
   - **R²**: Aminoalkylreste der allgemeinen Formel II

   -R³-NR⁴R⁵ (II),
   - **R³**: zweiwertige Kohlenwasserstoffreste mit 1-40 Kohlenstoffatomen,
   - **R⁴**: einwertige Kohlenwasserstoffreste mit 1-40 Kohlenstoffatomen oder H,
   - **R⁵**: einen Rest der allgemeinen Formel III

   -(R⁶-NR⁴)ₓR⁴ (III),
   - **R⁶**: einen zweiwertigen Rest der allgemeinen Formel IV

   - (CR⁴R⁴-)_{y} (IV),
   - **x**: den Wert 0 oder einen Wert von 1 bis 40,
   - **y**: den Wert 1 oder 2,
   - **a**: den Wert 0, 1, 2 oder 3,
   - **b**: den Wert 0, 1, 2 oder 3 und
   - **a+b**: einen durchschnittlichen Wert von 1,5 bis 2,5 bedeuten,
   wobei höchstens 9 mol% der Reste R¹ OH oder OR sind,
(ii) Protonierungsmittel
(iii) Wasser,
(iv) höchstens 5 Gew.-Teile Emulgator und
(v) MQ-Siliconharz enthalten, zur Behandlung von Leder und für die Papierleimung.

Die Emulsionen sind ohne weiteren Zusatz anderer stabilisierender Inhaltsstoffe, wie Emulgatoren oder Silicon-Polyether-Copolymer-Emulgatoren homogen, stabil und verdünnungsstabil. Sie enthalten vorzugsweise höchstens 3, besonders bevorzugt höchstens 1, insbesondere höchstens 0,1 Gew.-Teile Emulgator.

Die Alkylreste **R¹** und **R** können linear, zyklisch, verzweigt, gesättigt oder ungesättigt sein. Vorzugsweise weisen die Alkylreste **R¹** und **R** 1-18 Kohlenstoffatome, insbesondere 1 bis 6 Kohlenstoffatome auf, besonders bevorzugt sind Methylrest oder Ethylrest. Bevorzugte Halogensubstituenten sind Fluor und Chlor. Besonders bevorzugte Reste **R¹** sind Methylrest, Methoxirest, Ethoxirest oder -OH.

Die zweiwertigen Kohlenwasserstoffreste **R³** können halogensubstituiert, linear, zyklisch, verzweigt, aromatisch, gesättigt oder ungesättigt sein. Vorzugsweise weisen die Reste **R³** 1 bis 6 Kohlenstoffatome auf, besonders bevorzugt sind Alkylenreste, insbesondere Propylen. Bevorzugte Halogensubstituenten sind Fluor und Chlor.

Die einwertigen Kohlenwasserstoffreste **R⁴** können halogensubstituiert, linear, zyklisch, verzweigt, aromatisch, gesättigt oder ungesättigt sein. Vorzugsweise weisen die Reste **R⁴** 1 bis 6 Kohlenstoffatome auf, besonders bevorzugt sind Alkylreste. Bevorzugte Halogensubstituenten sind Fluor und Chlor. Besonders bevorzugte Substituenten **R⁴** sind Methyl, Ethyl, Cyclohexyl und H.

Vorzugsweise beträgt b den Wert 0 oder 1. Vorzugsweise beträgt a+b einen durchschnittlichen Wert von 1,9 bis 2,2.

Vorzugsweise weist **x** den Wert 0 oder einen Wert von 1 bis 18, besonders bevorzugt 1 bis 6 auf.

Besonders bevorzugte Reste **R²** sind -CH₂N(R⁴)₂, -(CH₂)₃N(R⁴)₂,-(CH₂)₃N(R⁴)(CH₂)₂N(R⁴)₂.

Vorzugsweise ist das Polydimethylsiloxan (P) aufgebaut aus mindestens 3, insbesondere mindestens 10 Einheiten der allgemeinen Formel I.

Das Verhältnis von a zu b ist so gewählt, daß das Polydimethylsiloxan (P) mindestens eine Aminzahl von 0,1 mequiv/g Polydimethylsiloxan (P), vorzugsweise mindestens 0,6 mequiv/g Polydimethylsiloxan (P) aufweist. Die Aminzahl des Polydimethylsiloxans (P) beträgt vorzugsweise höchstens 7 mequiv/g Polydimethylsiloxan (P).

Die Viskosität des Polydimethylsiloxans (P) beträgt vorzugsweise 1 bis 100000 mPa·s, insbesondere 10 bis 10000 mPa·s bei 25°C.

Vorzugsweise enthalten die Emulsionen Hilfsmittel, die ausgewählt werden aus Mono- oder Polyalkoholen und deren Ethern, welche einen Siedepunkt oder Siedebereich von höchstens 260 °C bei 0,10 MPa aufweisen.

Das Protonierungsmittel ist vorzugsweise eine einprotonige oder mehrprotonige, wasserlösliche oder wasserunlösliche, organische oder anorganische Säure, besonders bevorzugt Ameisensäure, Essigsäure, Schwefelsäure, Salzsäure oder Zitronensäure. Protonierungsmittel wird vorzugsweise in einer Menge von 0,05 bis 2 Mol Proton pro Mol basisches Stickstoffatom der Reste R² zugesetzt.

Das Wasser ist vollentsalztes oder salzhaltiges Wasser, bevorzugt vollentsalztes Wasser.

Vorzugsweise enthält das MQ-Siliconharz mindestens 80 Mol-%, vorzugsweise mindestens 95 Mol-%, Einheiten der allgemeinen Formel V und VI

R⁷₃SiO_{1/2} (V),

SiO_{4/2} (VI),

wobei
- **R⁷**: gegebenenfalls mit Halogenen substituierte Kohlenwasserstoffreste mit 1-40 Kohlenstoffatomen, Reste H, -OR oder -OH bedeuten,
das Verhältnis der Einheiten der allgemeinen Formeln V und VI 0,5 bis 2,0, bevorzugt 0,5 bis 1,5 beträgt und höchstens 3 Gew.-% bevorzugt höchstens 2,5 Gew.-% der Reste **R⁷** -OR und -OH sind.

Die restlichen Einheiten des MQ-Siliconharzes sind vorzugsweise Einheiten der allgemeinen Formel VII und VIII

R⁷₂SiO_{2/2} (VII),

R⁷SO_{3/2} (VIII).

Die einwertigen Kohlenwasserstoffreste **R⁷** können halogensubstituiert, linear, zyklisch, verzweigt, aromatisch, gesättigt oder ungesättigt sein. Vorzugsweise weisen die Reste **R⁷** 1 bis 6 Kohlenstoffatome auf, besonders bevorzugt sind Alkylreste und Phenylreste. Bevorzugte Halogensubstituenten sind Fluor und Chlor. Besonders bevorzugte Substituenten **R⁷** sind Methyl, Ethyl, Phenyl und H.
Vorzugsweise enthalten die Emulsionen 1 bis 200 Gew.-Teile, besonders bevorzugt 5 bis 100 Gew.-Teile MQ-Siliconharz.

Die Emulsionen können leicht hergestellt werden, durch Zusammengegeben und Vermischen der einzelnen Komponenten in beliebiger Reihenfolge.

Alle vorstehenden Symbole der vorstehenden Formeln weisen ihre Bedeutungen jeweils unabhängig voneinander auf. In allen Formeln ist das Siliciumatom vierwertig.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Beispiele

### Teil A

1. Um den Vorteil der erfindungsgemässen Emulsionen aufzuzeigen, wurden diese im Vergleich zu wässrigen, emulgatorhaltigen Zusammensetzungen und einer lösemittelbasierenden Zusammensetzung, jeweils auf Basis desselben Wirkstoffs, in Bezug auf ihre hydrophobierende Wirkung getestet.

Das in den Testbeispielen, verwendete aminofunktionelle Organopolysiloxan mit den funktionellen Gruppen -(CH₂)₃NH(CH₂)₂NH₂ weist eine Viskosität von ca. 1000 mm²/s bei 20 °C und eine Aminzahl von 0,6 mequiv/g Organopolysiloxan auf. Dieses Organopolysiloxan wird nachfolgend einfach als Aminöl bezeichnet.

### Herstellen der verschiedenen Zusammensetzungen:

### Erfindungsgemäße, wässrige Zusammensetzung A1 :

17 g einer Mischung aus 13,6 g Aminöl und 3,4 g MQ-Siliconharz werden unter Rühren bei Raumtemperatur zu 7 g Wasser, 7 g Ethylenglykolmonobutylether und 0,13 g Essigsäure gegeben, anschließend werden noch 68,87 g Wasser eingerührt. Erhalten wird eine milchig-opake Emulsion. Zum Anwendungstest wird die Emulsion auf einen Wirkstoffanteil von 5 % mit vollentsalztem Wasser verdünnt.

### Vergleichsbeispiel 3: Wässrige, emulgatorhaltige

### Zusammensetzung B1:

17 g einer Mischung aus 13,6 g Aminöl und 3,4 g MQ-Siliconharz werden unter Rühren bei Raumtemperatur zu 7 g Wasser, 7 g Emulgator (Fettalkoholethoxilat mit ca. 5 EO) und 0,13 g Essigsäure gegeben, anschließend werden noch 68,87 g Wasser eingerührt. Erhalten wird eine milchig-opake Emulsion. Zum Anwendungstest wird die Emulsion auf einen Wirkstoffanteil von 5 % mit vollentsalztem Wasser verdünnt.

### 1.1. Testung auf porösen Oberflächen

### 1.1.2. Anwendung auf Leder

Zur Testung der Imprägnierwirkung auf Leder wurde ein Lederfasermaterial verwendet, da dieses Material einheitlichere Oberflächen aufweist als Naturleder.

Die Testflüssigkeiten (5 %ig) läßt man mittels einer Pipette über die schräg gestellten Prüfmuster (ca. 45°) laufen, bis die Fläche benetzt ist. Anschließend läßt man die Proben über Nacht bei Raumtemperatur trocknen. Auf die flach ausgelegten Proben wird jeweils ein 0,04 ml großer Wassertropfen gesetzt und mit einem Glas abgedeckt, damit die Verdunstung des Wassertropfens die Messung nicht beeinflusst. Die Zeit bis zum vollständigen Einziehen des Tropfens wird gemessen. Es werden jeweils Doppelwerte bestimmt.

Als Ergebnis für das hydrophob ausgerüstete Leder wird die Eindringzeit des Tropfens angegeben, siehe Tabelle 5.

| **Tabelle 5** | unbehandelt | Zusammensetzung A1 | Zusammensetzung B1 |
|---|---|---|---|
| | | | Vergleichsbeispiel 3 |
| | | | |
| Eindringzeit [min] | 8 | 45 | 9 |

Die erfindungsgemäße Zusammensetzung A1 ist der herkömmlichen Zusammensetzung B1 in Bezug auf die Wassereindringzeit deutlich überlegen.

### Teil B

Beispiele zur Verwendung der Zusammensetzungen in der Papierleimung.

Um den Vorteil der wässrigen Zusammensetzungen für die Papierleimung aufzuzeigen, wurden diese im Vergleich zu wässerigen, emulgatorhaltigen Zusammensetzungen jeweils auf Basis desselben Wirkstoffs auf ihre Wirkung als Leimungsmittel getestet.

Lösemittelbasierende Zusammensetzungen wurden nicht zum Vergleich herangezogen, da deren Einsatz für die o. g. wässerigen Anwendungen nicht in Betracht kommen.

### Verwendete aminofunktionelle Organopolysiloxane:

### Aminöl 1:

Weist eine Viskosität von ca. 1000 mm²/s bei 25 °C, die funktionellen Reste (CH₂)₃NH(CH₂)₂NH₂ und eine Aminzahl von 0,6 mequiv./g Organopolysiloxan auf. Zudem enthält das Organopolysiloxan als Endgruppen ca. 0,75 Mol-% reaktive OMe/OH-Reste

### Aminöl 2:

Weist eine Viskosität von ca. 1000 mm²/s bei 25 °C, die funktionellen Reste -(CH₂)₃NH(CH₂)₂NH₂ und eine Aminzahl von 0,6 mequiv./g Organopolysiloxan auf.
Die Endgruppen sind in diesem Fall Me₃SiO-Reste.

### Aminöl 3:

Weist eine Viskosität von ca. 230 mm²/s bei 25 °C die funktionellen Reste -(CH₂)₃NH(CH₂)₂NH₂ und eine Aminzahl von 2,6 mequiv./g Organopolysiloxan auf. Die Endgruppen sind bei diesem Aminöl ebenfalls Me₃SiO-Reste.

### Aminöl 4:

Wird gemäß Beispiel 5 in US 2004/0029981 A1 hergestellt.
Das danach hergestellte Aminöl weist eine Viskosität von ca. 1.000 mm²/s bei 25°C, die funktionellen Reste -(CH₂)₃NH(CH₂)₃NH₂ und eine Aminzahl von 0,6 mequiv./g Organopolysiloxan auf.
Die Endgruppen sind in diesem Fall Me₃SiO-Reste.

### Herstellen der verschiedenen Zusammensetzungen:

### Wässrige Zusammensetzung A1:

16 g des Aminöls 1 werden unter Rühren bei Raumtemperatur zu 6 g Wasser,6 g Ethylenglykolmonobutylether und 0,17 g Essigsäure gegeben, anschließend wird das auf 100 g Gesamtgewicht fehlende Restwasser noch eingearbeitet. Erhalten wird eine milchig-opake Emulsion. Je nach Anwendungen wird auf 4 % bzw. 0,2 % Wirkstoffanteil mit voll entsalztem Wasser verdünnt.

### Wässriges, emulgatorhaltiges Vergleichsbeispiel B1:

16 g des Aminöls 1 werden unter Rühren bei Raumtemperatur zu 6 g Wässer, 6 g organischen Emulgator (Fettalkoholethoxyilat mit ca. 6 EO) und 0,17 g Essigsäure gegeben, anschließend wird noch das auf 100 g Gesamtgewicht fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Wässrige Zusammensetzung A2:

16 g einer Mischung aus 11,1 g Aminöl 1 und 4,9 g MQ-Harz werden unter Rühren bei Raumtemperatur zu 6 g Wasser, 6 g Ethylenglykolmonobutylether und 0,17 g Essigsäure gegeben, anschließend wird das auf ein Gesamtgewicht von 100 g noch fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Wässriges, emulgatorhaltiges Vergleichsbeispiel B2:

16 g einer Mischung aus 11,1 g Aminöl 1 und 4,9 g MQ-Harz werden unter Rühren bei Raumtemperatur zu 6 g Wasser, 6 g - organischen Emulgator (Fettalkoholethoxyilat mit ca. 6 EO) und 0,17 g Essigsäure gegeben, anschließend wird noch das auf 100 g Gesamtgewicht fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Wässrige Zusammensetzung C1:

16 g des Aminöls 2 werden unter Rühren bei Raumtemperatur zu 6 g Wasser und 6 g Ethylenglykolmonobutylether und 0,17 g Essigsäure gegeben, anschließend wird das auf 100 g Gesamtgewicht fehlende Restwasser noch eingearbeitet. Erhalten wird eine milchig-opake Emulsion. Je nach Anwendungen wird auf 4 % bzw. 0,2 % Wirkstoffanteil mit voll entsalztem Wasser verdünnt.

### Emulgatorhaltiges, wässriges Vergleichsbeispiel D1:

16 g des Aminöls 2 werden unter Rühren bei Raumtemperatur zu 6 g Wasser, 6 g organischen Emulgator (Fettalkoholethoxyilat mit ca. 6 EO) und 0,17 g Essigsäure gegeben, anschließend wird noch das auf 100 g Gesamtgewicht fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Wässrige Zusammensetzung C 2:

16 g einer Mischung aus 11,1 g Aminöl 2 und 4,9 g MQ-Harz werden unter Rühren bei Raumtemperatur zu 6 g Wasser, 6 g Ethylenglykolmonobutylether und 0,17 g Essigsäure gegeben, anschließend wird das auf ein Gesamtgewicht von 100 g noch fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Emulgatorhaltiges, wässriges Vergleichsbeispiel D2:

16 g einer Mischung aus 11,1 g Aminöl 2 und 4,9 g MQ-Harz werden unter Rühren bei Raumtemperatur zu 6 g Wasser, 6 g organischen Emulgator (Fettalkoholethoxyilat mit ca. 6 EO) und 0,17 g Essigsäure gegeben, anschließend wird noch das auf 100 g Gesamtgewicht fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Wässrige Zusammensetzung E1 :

16 g des Aminöls 3 werden unter Rühren bei Raumtemperatur zu 6 g Wasser und 0,17 g Essigsäure gegeben, anschließend wird das auf 100 g Gesamtgewicht fehlende Restwasser noch eingearbeitet. Erhalten wird eine milchig-opake Emulsion. Je nach Anwendungen wird auf 4 % bzw. 0,2 % Wirkstoffanteil mit voll entsalztem Wasser verdünnt.

### Emulgatorhaltiges, wässriges Vergleichsbeispiel F1:

16 g des Aminöls 3 werden unter Rühren bei Raumtemperatur zu 6 g Wasser, 6 g organischen Emulgator (Fettalkoholethoxyilat mit ca. 6 EO) und 0,17 g Essigsäure gegeben, anschließend wird noch das auf 100 g Gesamtgewicht fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Wässrige Zusammensetzung E2:

16 g einer Mischung aus 11,1 g Aminöl 3 und 4,9 g MQ-Harz werden unter Rühren bei Raumtemperatur zu 6 g Wasser und 0,17 g Essigsäure gegeben, anschließend wird das auf ein Gesamtgewicht von 100 g noch fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Emulgatorhaltiges, wässriges Vergleichsbeispiel F2:

16 g einer Mischung aus 11,1 g Aminöl 3 und 4,9 g MQ-Harz werden unter Rühren bei Raumtemperatur zu 6 g Wasser, 6 g organischen Emulgator (Fettalkoholethoxyilat mit ca. 6 EO) und 0,17 g Essigsäure gegeben, anschließend wird noch das auf 100 g Gesamtgewicht fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Wässriges Vergleichsbeispiel G1:

16 g des Aminöls 4 werden unter Rühren bei Raumtemperatur zu 6 g Wasser und 6 g Ethylenglykolmonobutylether und 0,17 g Essigsäure gegeben, anschließend wird noch das auf 100 g Gesamtgewicht fehlende Restwasser eingearbeitet. Erhalten wird eine milchig-opake Emulsion. Je nach Anwendung wird auf 4 % bzw. 0,2 % Wirkstoffanteil mit voll entsalztem Wasser verdünnt.

### Wässriges Vergleichsbeispiel G2:

16 g einer Mischung aus 11,1 g Aminöl 4 und 4,9 g MQ-Harz werden unter Rühren bei Raumtemperatur zu 6 g Wasser, 6 g Ethylenglykolmonobutylether und 0,17 g Essigsäure gegeben, anschließend wird das auf 100 g Gesamtgewicht fehlende Restwasser noch eingearbeitet. Erhalten wird eine milchig-opake Emulsion. Je nach Anwendung wird auf 4 % bzw. 0,2 % Wirkstoffanteil mit voll entsalztem Wasser verdünnt.

### Prüfung der Leimungswirkung auf Papier

### Behandlung des ungeleimten Papiers:

Im Labor gelagerte, ungeleimte Prüfpapiere werden zweimal je 5 s (Vorder- und Rückseite je 5 s) auf eine auf 0,2 % Wirkstoffgehalt verdünnte Leimungsflotte gelegt, so dass das Prüfpapier vollständig mit Leimungsflotte getränkt ist.
Danach wird das mit Leimungsflotte getränkte Papier zwischen zwei Gummiwalzen geschoben, um überschüssige Leimungslösung abzuquetschen. Sofort danach wird das nasse abgequetschte Papier zurückgewogen und aus der Differenzmasse die Auftragsmenge an Leimungsflotte bzw. Wirkstoff berechnet. Es erfolgt Trocknung für 3 Minuten bei 150 °C im Umlufttrockenschrank, und zum Glätten des Papiers wird das Papier mit einem Bügeleisen der Marke Rowenta^{®} bei Stufe 2,5 gebügelt.

### Prüfung auf Leimung:

Nach dem Bügeln wird das Papier nach 2 Tagen Lagerung bei Raumtemperatur auf Leimung geprüft. Hierzu werden aus den geleimten Blättern Stücke von 8 x 8 cm² herausgeschnitten, an den Enden hochgebogen und anschließend auf eine Tintenlösung (stark konzentrierte grüne Schreibertinte) gelegt. Nun misst man mit der Stoppuhr die Zeit, bis 5 % der Oberfläche des Prüfpapiers mit durchgeschlagener Prüftinte bedeckt ist. Je größer die Zeitdauer umso besser die Leimung des Papiers. Für die Werte in der nachstehenden Tabelle wurden 0,22 Gew.-% an Wirkstoff (Aminöl plus gegebenenfalls MQ-Harz) bezogen auf Papiermasse aufgetragen.

Ergebnisse siehe Tabelle 9.

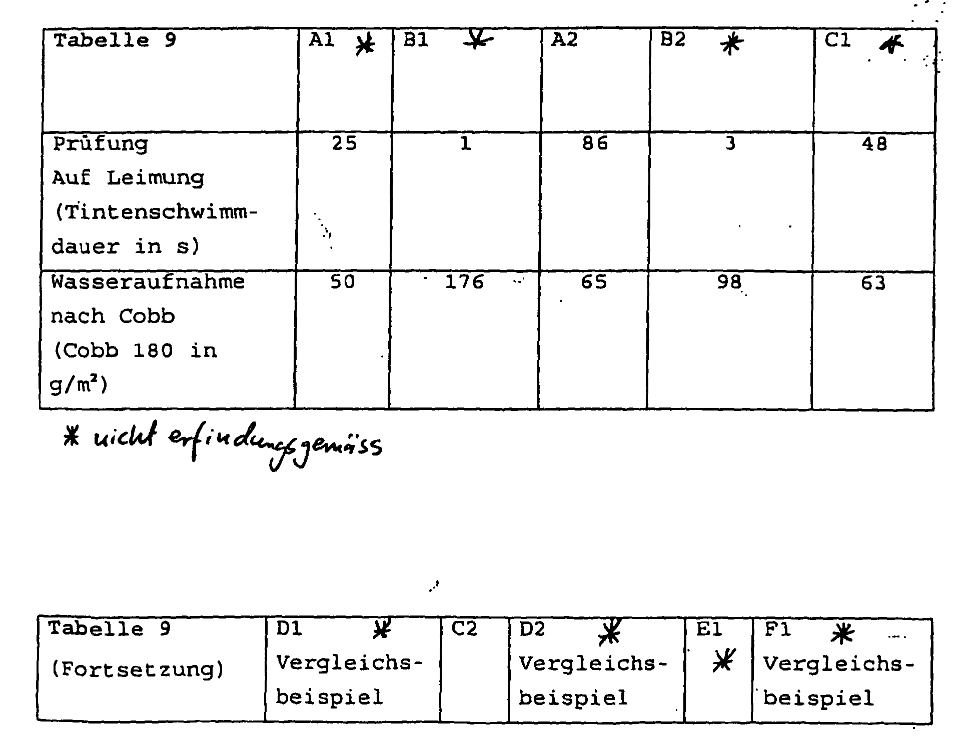

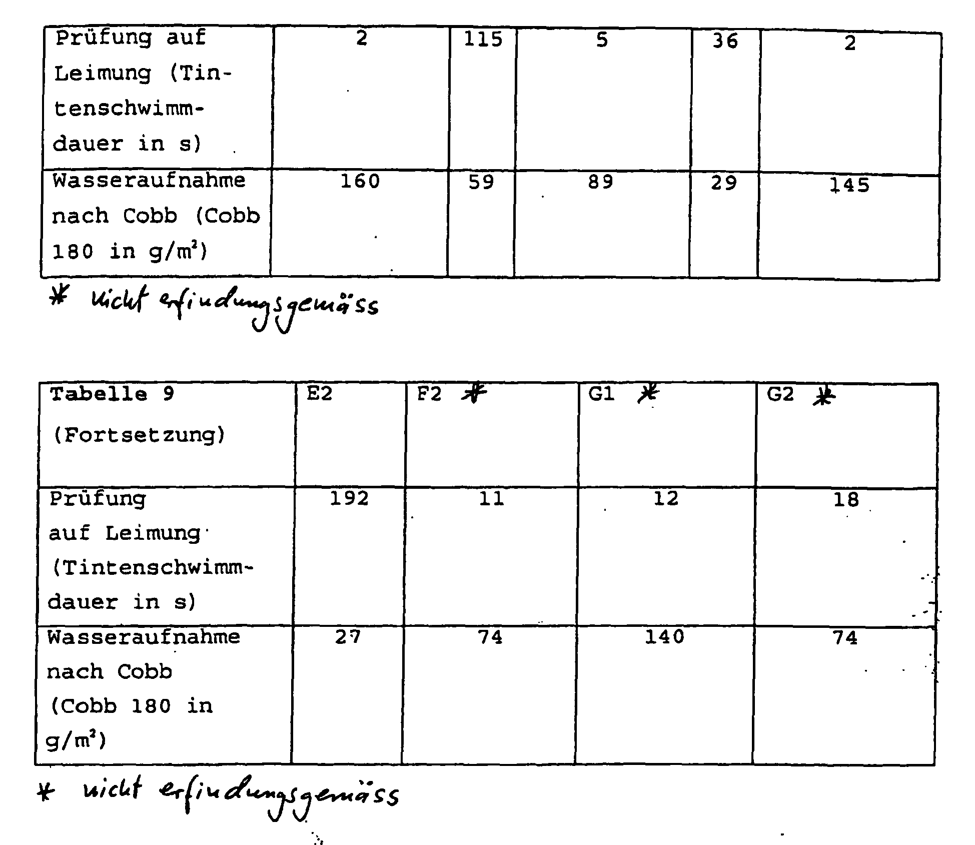

## Patentansprüche

1. Verwendung von Öl-in-Wasser Emulsionen, die
(i) 100 Gew.-Teile eines Aminoalkylgruppen aufweisenden Polydimethylsiloxans (P) mit einer Aminzahl von mindestens 0,1 mequiv/g Polydimethylsiloxan (P), aufgebaut aus Einheiten der allgemeinen Formel I
R¹ₐR²_{b}SiO_{(4-a-b/2)} (I),
in der
**R¹** gegebenenfalls mit Halogenen substituierte Alkylreste mit 1-40 Kohlenstoffatomen, Reste -OR oder -OH,
**R** gegebenenfalls mit Halogenen substituierte Alkylreste mit 1-40 Kohlenstoffatomen,
**R²** Aminoalkylreste der allgemeinen Formel II
-R³-NR⁴R⁵ (II),
**R³** zweiwertige Kohlenwasserstoffreste mit 1-40 Kohlenstoffatomen,
**R⁴** einwertige Kohlenwasserstoffreste mit 1-40 Kohlenstoffatomen oder H,
**R⁵** einen Rest der allgemeinen Formel III
-(R⁶-NR⁴)ₓR⁴ (III),
**R⁶** einen zweiwertigen Rest der allgemeinen Formel IV
-(CR⁴R⁴-)_{y} (IV),
**x** den Wert 0 oder einen Wert von 1 bis 40,
**y** den Wert 1 oder 2,
**a** den Wert 0, 1, 2 oder 3,
**b** den Wert 0, 1, 2 oder 3 und
**a+b** einen durchschnittlichen Wert von 1,5 bis 2,5 bedeuten,
wobei höchstens 9 mol% der Reste **R¹** OH oder O**R** sind,
(ii) Protonierungsmittel
(iii) Wasser,
(iv) höchstens 5 Gew.-Teile Emulgator und
(v) MQ-Siliconharz enthalten, zur Behandlung von Leder und für die Papierleimung.

2. Verwendung nach Anspruch 1, bei der **R⁴** Alkylreste mit 1 bis 6 Kohlenstoffatomen bedeutet.

3. Verwendung nach Anspruch 1 oder 2, bei denen die Reste **R²** ausgewählt werden aus -CH₂N(R⁴)₂, -(CH₂)₃N(R⁴)₂, und -(CH₂)₃N(R⁴)(CH₂)₂N(R⁴)₂.

4. Verwendung nach Anspruch 1 bis 3, bei der die Emulsionen Hilfsmittel enthalten, die ausgewählt werden aus Mono- oder Polyalkoholen und deren Ethern, welche einen Siedepunkt oder Siedebereich von höchstens 260 °C bei 0,10 MPa aufweisen.

5. Verwendung nach Anspruch 1 bis 4, bei denen das Protonierungsmittel ausgewählt wird aus Ameisensäure, Essigsäure, Schwefelsäure, Salzsäure und Zitronensäure.

6. Verwendung nach Anspruch 1 bis 5, bei der die Emulsionen 1 bis 200 Gew.-Teile MQ-Siliconharz enthalten.

## Claims

1. Use of oil-in-water emulsions which contain
(i) 100 parts by weight of a polydimethylsiloxane (P) having aminoalkyl groups and having an amine number of at least 0.1 meq/g of polydimethylsiloxane (P), composed of units of the general formula I
R¹ₐR²_{b}SiO_{(4-a-b/2)} (I),
in which
**R¹** are optionally halogen-substituted alkyl radicals having 1-40 carbon atoms, or -OR or -OH radicals,
**R** are optionally halogen-substituted alkyl radicals having 1-40 carbon atoms,
**R²** are aminoalkyl radicals of the general formula II
-R³-NR⁴R⁵ (II),
**R³** are divalent hydrocarbon radicals having 1-40 carbon atoms,
**R⁴** are monovalent hydrocarbon radicals having 1-40 carbon atoms or H,
**R⁵** is a radical of the general formula III
-(R⁶-NR⁴)ₓR⁴ (III),
**R⁶** is a divalent radical of the general formula IV
-(CR⁴R⁴-)_{y} (IV),
**x** has the value 0 or a value from 1 to 40,
**y** has the value 1 or 2,
**a** has the value 0, 1, 2 or 3,
**b** has the value 0, 1, 2 or 3 and
**a+b** has an average value of from 1.5 to 2.5,
not more than 9 mol% of the radicals **R¹** being OH or OR,
(ii) protonating agents,
(iii) water,
(iv) not more than 5 parts by weight of emulsifier and
(v) MQ silicone resin, for the treatment of leather and for the sizing of paper.

2. Use according to Claim 1, in which **R⁴** are alkyl radicals having 1 to 6 carbon atoms.

3. Use according to Claim 1 or 2, in which the radicals **R²** are selected from -CH₂N(R⁴)₂, -(CH₂)₃N(R⁴)₂, and-(CH₂)₃N(R⁴)(CH₂)₂N(R⁴)₂.

4. Use according to any of Claims 1 to 3, in which the emulsions contain auxiliaries which are selected from mono- or polyalcohols and ethers thereof, which have a boiling point or boiling range of not more than 260°C at 0.10 MPa.

5. Use according to any of Claims 1 to 4, in which the protonating agent is selected from formic acid, acetic acid, sulfuric acid, hydrochloric acid and citric acid.

6. Use according to any of Claims 1 to 5, in which the emulsions contain from 1 to 200 parts by weight of MQ silicone resin.

## Revendications

1. Utilisation d'émulsions huile/eau qui comprennent:
(i) 100 parties en poids d'un polydiméthylsiloxane renfermant des groupes aminoalkyle (P), présentant un indice d'amine d'au moins 0,1 méquiv/g de polydiméthylsiloxane (P), constitué de motifs de formule générale I :
R¹ₐR²_{b}SiO_{(4-a-b/2)} (I),
dans laquelle:
**R¹** représente des radicaux alkyle éventuellement substitués par des atomes d'halogène, renfermant de 1 à 40 atomes de carbone, des radicaux -OR ou -OH,
**R** représente des radicaux alkyle éventuellement substitués par des atomes d'halogène, renfermant de 1 à 40 atomes de carbone,
**R²** représente des radicaux aminoalkyle de formule générale II
-R³-NR⁴R⁵ (II),
**R³** représente des radicaux hydrocarbonés bivalents renfermant de 1 à 40 atomes de carbone,
**R⁴** représente des radicaux hydrocarbonés monovalents renfermant de 1 à 40 atomes de carbone ou un atome d'hydrogène,
**R⁵** représente un radical de formule générale III
-(R⁶-NR⁴)ₓR⁴ (III),
**R⁶** représente un radical bivalent de formule générale IV
-(CR⁴R⁴-)_{y} (IV),
**x** vaut 0 ou vaut de 1 à 40,
**y** vaut 1 ou 2,
**a** vaut 0, 1, 2 ou 3,
**b** vaut 0, 1, 2 ou 3, et
**a+b** vaut en moyenne de 1,5 à 2,5, où au plus 9 % en moles des radicaux **R¹** sont des groupes OH ou OR,
(ii) un agent de protonation,
(iii) de l'eau,
(iv) au plus 5 parties en poids d'un agent émulsifiant, et
(v) une résine siliconée MQ, pour le traitement du cuir et pour le collage du papier.

2. Utilisation selon la revendication 1, dans laquelle **R⁴** représente des radicaux alkyle renfermant de 1 à 6 atomes de carbone.

3. Utilisation selon la revendication 1 ou 2, dans laquelle les radicaux **R²** sont choisis parmi un groupe -CH₂N(R⁴)₂, un groupe -(CH₂)₃N(R⁴)₂ et un groupe -(CH₂)₃N(R⁴) (CH₂)₂N(R⁴)₂

4. Utilisation selon les revendications 1 à 3, dans laquelle les émulsions comprennent des auxiliaires choisis parmi des mono- ou des polyalcools et leurs éthers, qui présentent un point d'ébullition ou une plage d'ébullition d'au plus 260 °C à 0,10 MPa.

5. Utilisation selon les revendications 1 à 4, dans laquelle l'agent de protonation est choisi parmi l'acide formique, l'acide acétique, l'acide sulfurique, l'acide chlorhydrique et l'acide citrique.

6. Utilisation selon les revendications 1 à 5, dans laquelle les émulsions comprennent de 1 à 200 parties en poids d'une résine siliconée MQ.
